Europäisches Patentamt

European Patent Office

Office européen des brevets

(11) Veröffentlichungsnummer: **0 113 653**
**B1**

## (12) EUROPÄISCHE PATENTSCHRIFT

(45) Veröffentlichungstag der Patentschrift:
**07.10.87**

(21) Anmeldenummer: **83890219.5**

(22) Anmeldetag: **05.12.83**

(51) Int. Cl.⁴: **G 01 N 1/12, C 21 C 5/46**

(54) Vorrichtung zum Wechseln von Mess- und/oder Probenahmesonden.

(30) Priorität: **09.12.82 AT 4474/82**

(43) Veröffentlichungstag der Anmeldung:
**18.07.84 Patentblatt 84/29**

(45) Bekanntmachung des Hinweises auf die Patenterteilung:
**07.10.87 Patentblatt 87/41**

(84) Benannte Vertragsstaaten:
**BE DE FR GB IT LU NL SE**

(56) Entgegenhaltungen:
**EP - A - 0 036 912**
**DE - A - 3 044 609**
**FR - A - 2 410 236**
**GB - A - 2 014 728**

(73) Patentinhaber: **VOEST-ALPINE Aktiengesellschaft,**
**Muldenstrasse 5, A-4020 Linz (AT)**

(72) Erfinder: **Fohler, Johann, Efeugang 10, A-4040 Puchenau**
**(AT)**

(74) Vertreter: **Wolfram, Gustav, Dipl.-Ing.,**
**Schwindgasse 7 P.O. Box 205, A-1041 Wien (AT)**

## Beschreibung

Die Erfindung betrifft eine Vorrichtung zum Wechseln von Mess- und/oder Probeentnahmesonden, die auf eine am unteren Ende einer vertikal beweglichen Lanze angeordnete Halterung unter Reibschlusskontakt aufschiebbar sind, mit einem die Sonde festklemmenden, an einem Arm der Vorrichtung angeordneten Greifer, der mittels des Arms aus einer Arbeitsposition unterhalb der Lanze in eine seitlich daneben befindliche Position bringbar ist.

Eine Einrichtung dieser Art ist z.B. aus der DE-A-3 044 609 bekannt. Bei Durchführung des Messvorganges bzw. der Probeentnahme durch Eintauchen der Sonde in ein eine zu beobachtende Schmelze enthaltendes Gefäss, beispielsweise in einen Stahlwerkskonverter, kann es entweder durch Auffahren der Sonde auf noch nicht aufgeschmolzenes Gut bzw. durch Anstossen der Sonde mit auf dem schmelzflüssigen Gut schwimmenden Schlackenteilen zu einem Verbiegen der die Sonde tragenden Halterung kommen. Nach Rückziehen der Lanze nimmt dann die Sonde eine von der senkrechten Ideallage abweichende Schräglage ein, wodurch sie von dem Greifer nicht mehr sicher erfasst werden kann.

Eine besondere Problematik ergibt sich beim Aufschieben der nächsten Sonde mittels des Greifers, da die Sonde vom Greifer in genau vertikaler Lage möglichst starr – zur Aufnahme der Schiebekräfte beim Aufschieben der Sonde auf die Halterung – gehalten wird. Beim Aufschieben auf die Halterung der Lanze, welches in der Regel durch Absenken der Lanze bewerkstelligt wird, kann es infolge unterschiedlicher Lagen der Achse der Halterung und der Achse der Sonde zu einer Beschädigung oder Zerstörung der Sonde durch die Halterung kommen. Für Messsonden ist die Halterung als tief in die Sonde eindringender Haltestab ausgebildet, der nahe seinem unteren Ende mit Kontaktstellen zwecks Anschluss an die in der Sonde vorgesehenen Messeinrichtungen versehen ist. Beim Aufschieben der Sonde auf den Haltestab kann es bei nicht fluchtender Achslage von Sonde und Haltestab zu einer Beschädigung der Kontaktstellen kommen, wodurch Fehlmessungen verursacht werden können bzw. die Durchführung einer Messung nicht mehr möglich ist.

Die Erfindung bezweckt die Vermeidung dieser Nachteile und Schwierigkeiten und stellt sich die Aufgabe, eine Vorrichtung der eingangs beschriebenen Art zu schaffen, die ein sicheres Aufschieben einer Sonde auf eine hinsichtlich ihrer Lage eine von der Idealposition abweichende Position einnehmende Halterung ermöglicht, wobei Beschädigungen der Halterung und der Sonden vermieden werden und wobei die Sonden nach Durchführung der Messung bzw. nach Probenahme vom Greifer sicher erfasst und von der Lanze einwandfrei abgezogen werden können.

Diese Aufgabe wird erfindungsgemäss dadurch gelöst, dass der Greifer an der Vorrichtung kardanisch gelagert ist. Durch die kardanische Lagerung des Greifers kann sich der Greifer bei Kontakt mit der Messsonde bzw. bei Kontakt der Sonde mit der Halterung selbsttätig an die Lage der Sonde bzw. die Lage der Halterung anpassen.

Zweckmässig liegt der Schnittpunkt der Achsen der kardanischen Lagerung bei einem in Arbeitsposition befindlichen Greifer in der Achse der Lanze.

Gemäss einer bevorzugten Ausführungsform ist der Greifer aus seiner Ruhelage heraus gegen Rückstellkräfte kardanisch verschwenkbar, wobei die Rückstellkräfte entweder durch elastische Mittel oder durch Anordnung des Schwerpunktes des Greifers unterhalb des Schnittpunktes der Achsen der kardanischen Lagerung hervorgerufen ist, wodurch der Greifer nach einem Greifvorgang bzw. nach dem Aufschieben einer Sonde auf die Halterung stets selbsttätig in seine Ausgangslage zurückkehrt.

Damit der Greifer auch während des Aufschiebens, insbesondere zu Beginn des Aufschiebens, eine stabile Lage einnimmt, gelangt die Halterung im Abstand unterhalb des Schnittpunktes der Achsen der kardanischen Lagerung mit der Sonde in Reibschlusskontakt.

Kommt es zu einer besonders grossen Abweichung der Lage der Halterung von der Ideallage, ist es möglich, dass das untere in die Sonde einschiebbare Ende der Halterung bei Aufschieben der Sonde auf die Halterung ausserhalb der Öffnung der Sonde auf die Sonde auftritt oder neben der Sonde vorbeigeführt wird. Um dies zu verhindern, ist gemäss einer bevorzugten Ausführungform bei einer Vorrichtung der eingangs beschriebenen Art eine in der europäischen Patentanmeldung 83 8 90 222.9, veröffentlicht unter der Nummmer 0 115 757, beschriebene und im Abstand oberhalb des in Arbeitsposition befindlichen Greifers angeordnete Zentriereinrichtung vorgesehen, die zwei Fangarme aufweist, die aus einer Ruheposition seitlich der Halterung in eine die Halterung fluchtend oberhalb des in Arbeitsposition befindlichen Greifers festlegende Fangposition bewegbar sind.

Eine Zentriereinrichtung ist aus der DE-B-2 753 161 bekannt. Diese bekannte Zentriereinrichtung ermöglicht die Einstellung des unteren Endes der Halterung bzw. der Lanze vertikal über der Sondenöffnung. Als Zentriereinrichtung sind Tastorgane vorgesehen, die die Lagekoordinaten der Halterung bzw. der auf die Halterung aufgeschobenen Sonde ermitteln, worauf in Abhängigkeit von den festgestellten Lagekoordinaten der Greifer zu diesen Lagekoordinaten verfahren wird. Ein Nachteil dieser bekannten Einrichtung ist darin zu sehen, dass eine relativ aufwendige Koordinatensteuerung mit ebenso komplizierten Steuerungsorganen für die den Greifer in die gewünschte Position verfahrenden Antriebe notwendig sind.

Gemäss der erfindungsgemässen Vorrichtung und mit Hilfe der Zentriereinrichtung wird der Greifer zum Aufschieben bzw. Abziehen der Sonden stets in dieselbe Position verfahren und wird die Lanze, deren unteres Ende ausreichend seitlich bewegbar ist (infolge der Aufhängung der Lanze an ihrem oberen Ende), in eine zum Greifer vertikal fluchtende Position bewegt.

Vorzugsweise sind die Fangarme als übereinanderliegende sichelförmige Arme ausgebildet und aus einer geöffneten Ruheposition in eine die Halterung umschliessende Fangposition schwenkbar, und zwar unter Verkleinerung des von den Fangarmen umschlossenen Raumquerschnittes bis auf den Querschnitt der Halterung.

Zweckmässig ist die Zentriereinrichtung an einem den Greifer bewegenden Arm montiert.

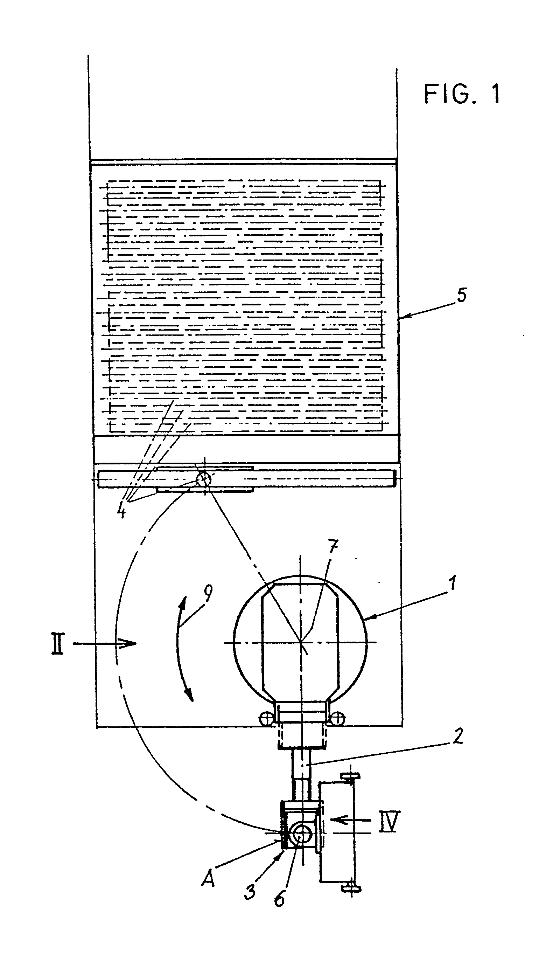

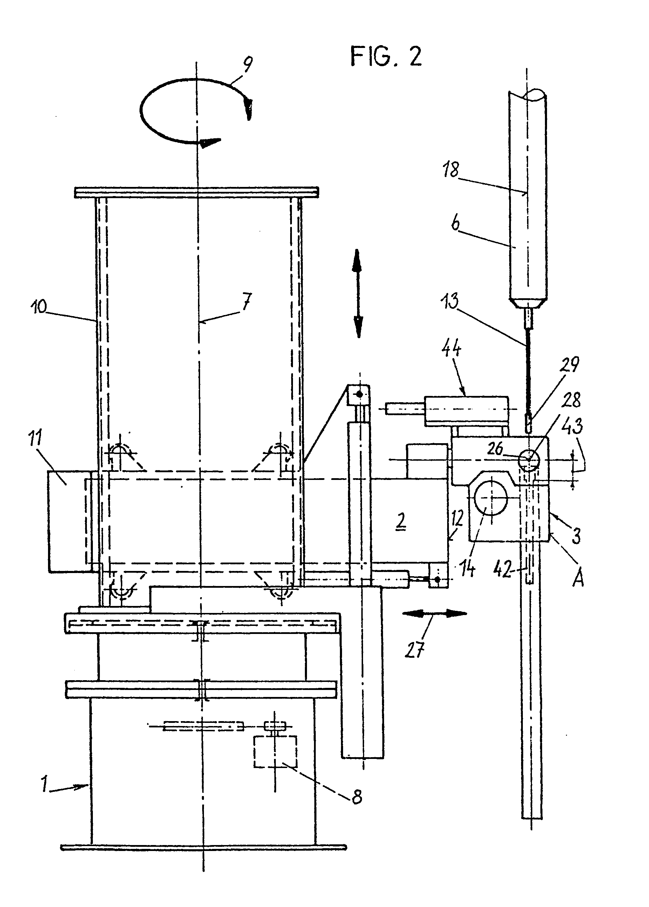

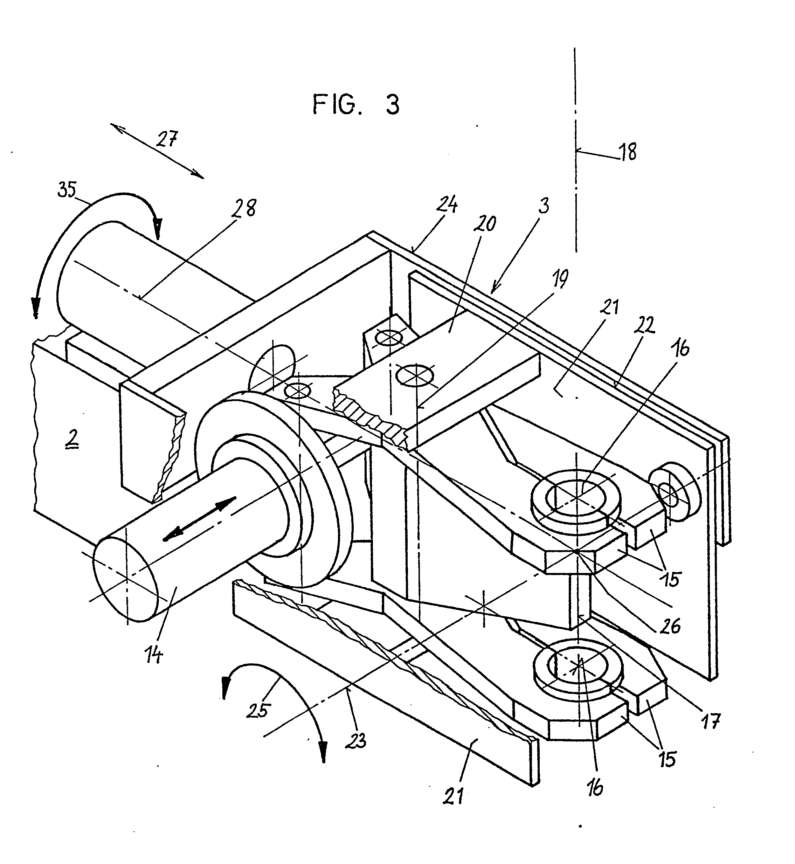

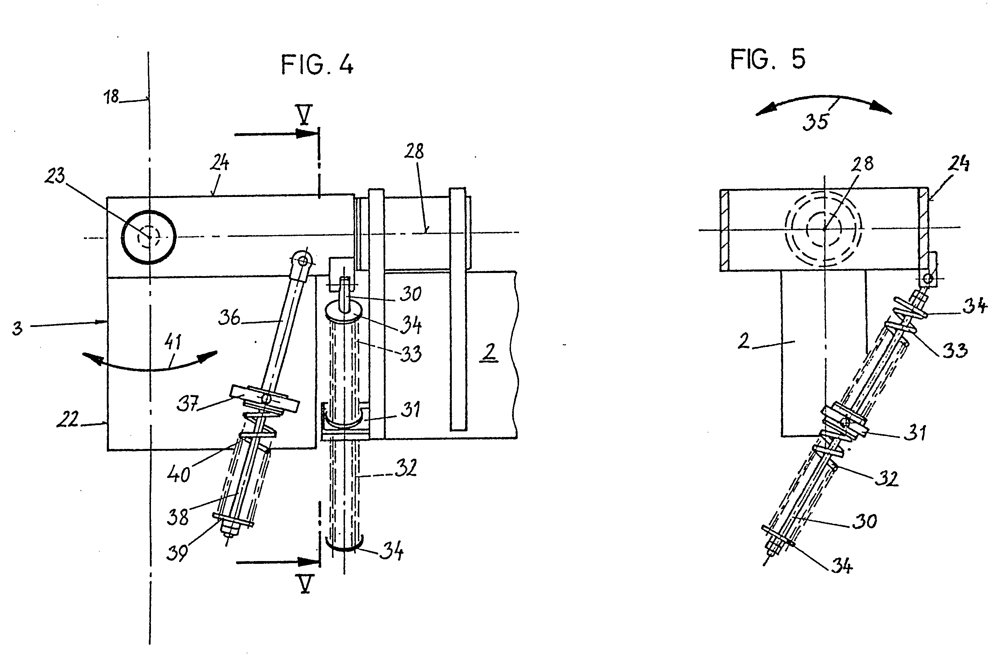

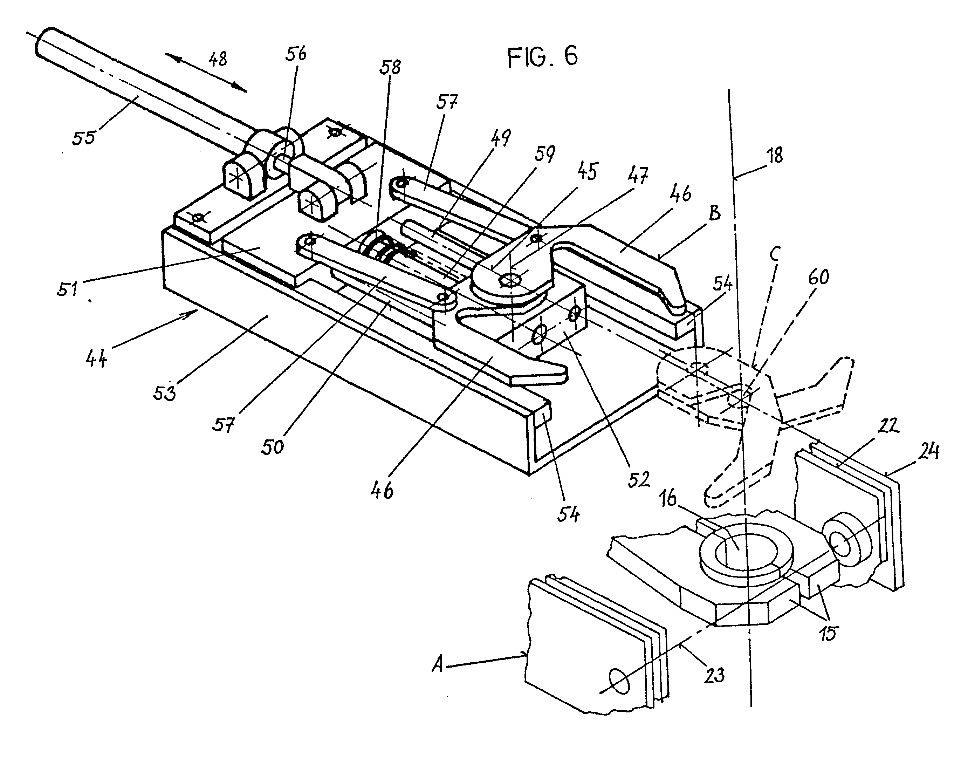

Die Erfindung ist nachfolgend anhand der Zeichnung an einem Ausführungsbeispiel näher erläutert, wobei Fig. 1 eine Draufsicht auf eine in einem Stahlwerk aufgestellte Vorrichtung zum Wechseln von Mess- und/oder Probenahmesonden veranschaulicht. Fig. 2 zeigt eine Ansicht in Richtung des Pfeiles II der Fig. 1 und Fig. 3 einen teilweise geschnittenen Schrägriss des Greifers. Fig. 4 zeigt eine Ansicht in Richtung des Pfeiles IV der Fig. 1, Fig. 5 einen Schnitt gemäss der Linie V–V der Fig. 4. Fig. 6 veranschaulicht einen Schrägriss einer Zentriereinrichtung.

Mit 1 ist ein Sondenmanipulator bezeichnet, der einen an einem Arm 2 befestigten Greifer 3 zur Aufnahme einer Sonde 4 aus einem Magazin 5 und überbringen dieser Sonde 4 zu einer Lanze 6 bzw. zum Abnehmen der Sonde 4 von der Lanze 6 aufweist. Der Manipulator weist eine um eine vertikale Achse 7 mittels eines Antriebes 8 in Richtung des Doppelpfeiles 9 drehbare Säule 10 auf, an der ein Wagen 11 in vertikaler Richtung verschiebbar gelagert ist. Dieser Wagen 11 dient zur Aufnahme des in horizontaler Richtung verschiebbaren Armes 2, an dessen freiem Ende 12 der die Sonde 4 festklemmende Greifer 3 montiert ist. Wie aus Fig. 1 ersichtlich ist, kann die Sonde 4 mittels des Greifers 3 dem Magazin 5 durch Schwenken um die Achse 7 entnommen werden und in eine Position fluchtend unterhalb der Lanze 6 gebracht werden. Durch Absenken der Lanze 6 wird die Sonde auf die an der Lanze montierte Halterung, die als Haltestab 13 ausgebildet ist, aufgeschoben. Zum Entfernen der Sonde 4 von der Lanze 6 wird nach Durchführen der Messung bzw. nach Probenahme die Sonde 4 mittels des Greifers 3 festgeklemmt und die Lanze 6 nach oben gezogen, wodurch die Sonde 4 vom Haltestab 13 abgezogen wird, worauf die Sonde 4 mittels des Greifers 3 zu einer Abgabestelle gebracht wird.

Der Greifer weist zwei vertikal übereinanderliegende, mittels eines Druckmittelzylinders 14 zu öffnende und zu schliessende Zangenbackenpaare 15 auf, zwischen deren fluchtenden kreisförmigen Ausnehmungen 16 eine Messsonde 4 festklemmbar ist. Die durch die kreisförmigen Ausnehmungen gelegte Achse, die mit der Achse 18 der Lanze 6 bei in Arbeitsposition A gebrachtem Greifer 3 fluchtet, ist mit 17 bezeichnet. Die Lagerachse 19 der Zangenbackenpaare 15 ist an Querträgern 20 befestigt, die gemeinsam mit vertikalen Platten 21 einen Innenrahmen 22 bilden. Der Innenrahmen 22 ist um eine etwa horizontal verlaufende Schwenkachse 23 gegenüber einem den Innenrahmen umgebenden Aussenrahmen 24 verschwenkbar gelagert, wodurch die Zangenbackenpaare 15 bzw. die durch die kreisförmigen Ausnehmungen 16 gelegte Achse 17 gegenüber dem Aussenrahmen 24 in Richtung des Doppelpfeiles 25 schwenkbar sind.

Die den Innenrahmen 22 mit dem Aussenrahmen verbindende Schwenkachse 23 schneidet die durch die kreisförmigen Ausnehmungen 16 gelegte Achse 17 in einem Punkt 26. Der Aussenrahmen 24 ist an dem Arm 2 um eine parallel zur Verschieberichtung 27 des Armes 2 angeordnete horizontale Achse 28, die zur den Aussenrahmen 24 mit dem Innenrahmen 22 verbindenden Schwenkachse 23 etwa rechtwinkelig angeordnet ist, verschwenkbar. Diese Achse 28 schneidet sich mit der den Innen- mit dem Aussenrahmen verbindenden Schwenkachse 23 in dem Punkt 26, in dem diese von der durch die kreisförmigen Ausnehmungen 16 gelegte Achse 17 geschnitten wird.

Die Achsen 23 und 28 bilden eine kardanische Aufhängung des Greifers 3, so dass die durch die kreisförmigen Ausnehmungen 16 gelegte Achse 17 in jede Richtung des Raumes ausgerichtet werden kann. Der Greifer 3 kann sich somit selbständig an einen schräg im Raum liegenden Haltestab 13 der Lanze 6, auf den die Sonde 4 aufgeschoben werden soll, ausrichten, so dass eine Beschädigung der an der Haltestange vorgesehenen Kontakte 29 vermieden wird und ein einwandfreier Sitz der Sonde 4 an dem Haltestab 13 gewährleistet ist. Beschädigungen der Sonde 4 werden vermieden.

Damit der Greifer 3 im Ruhezustand, d.h. ohne Einwirkung von Kräften, die von der Lanze herrühren, stets eine stabile Normalposition einnimmt, ist der Aussenrahmen 24 gegenüber dem Arm 2 mittels einer in einer Ebene senkrecht zur Achse 28 liegenden Zugstange 30 (Fig. 4 und 5) abgestützt, die einerseits am Aussenrahmen 24 und andererseits mittels eines Gleitringes 31 am Arm angelenkt ist. An beiden Seiten des Gleitringes 31 stützen sich unter Vorspannung stehende Schraubenfedern 32, 33 ab, die jeweils mit ihren dem Gleitring gegenüberliegenden Enden an der Zugstange 30 befestigten Stützplatten 34 abgestützt sind.

Aus Fig. 5 ist die Normalposition des Aussenrahmens 24 gegenüber dem Arm 2 ersichtlich. Sobald der Aussenrahmen 24 um die Achse 28 gegenüber dem Arm 2 in einer der Richtungen des Doppelpfeiles 35 ausgelenkt wird, wird eine der Federn 32, 33 komprimiert und die andere Feder dehnt sich aus. Die dabei auftretenden ungleichen Federkräfte wirken als Rückstellkräfte und trachten, nach einer Auslenkung den Aussenrahmen 24 wieder in seine ursprüngliche Normalposition zurückzuführen.

In Fig. 4 ist die Abstützung des Aussenrahmens 24 gegenüber dem Innenrahmen 22 veranschaulicht, wobei wiederum eine Zugstange 36 einerseits am Aussenrahmen 24 und andererseits mittels eines Gleitringes 37 am Innenrahmen 22 befestigt ist. An einer Verlängerung 38 der Zugstange stützt sich an einer Stützplatte 39 eine Schraubenfeder 40 ab, deren gegenüberliegendes Ende am Gleitring abgestützt ist. Sobald der Innenrahmen 22 in einer der Richtungen des Doppelpfeiles 41 gegenüber dem Aussenrahmen 24 verschwenkt wird, trachtet die dabei entweder zusammengedrückte oder entspannte Feder 40, den Innenrahmen wiederum in die Normalposition, in der die Feder 40 gerade den vom Eigengewicht des Innenrahmens 22 samt Zangenbackenpaaren 15 herrührenden Drehmoment das Gleichgewicht hält, zurückzuführen. Anstelle der den Greifer 3 in die Normalposition zurückschwenkenden Federn 32, 33, 40 könnte der Greifer 3 auch so konstruiert sein, dass der gemeinsame Schwenkpunkt von Aussenrahmen 24, Innenrahmen 22 und Zangenbackenpaaren 15 (samt Druckmittelzylinder 14) unterhalb des Schnittpunktes 26 der Achsen 23 und 28 der kardanischen Lagerung zu liegen kommt. In diesem Fall wäre die Rückführung des Greifers 3 in seine Normalposition durch das Eigengewicht des Greifers gewährleistet.

Damit beim Einfädeln des Haltestabes 13 in die Öffnung 42 der Sonde 4 keine unerwünschte Auslenkung der Sonde 4 bzw. der durch die kreisförmigen Ausnehmungen 16 gelegten Achse stattfindet, ist der Greifer 3 so ausgelegt, dass der Haltestab 13 mit der Sonde im Abstand 43 unterhalb des Schnittpunktes 26 der Achsen 23 und 28 der kardanischen Lagerung in Reibschlusskontakt gelangt.

Durch einseitige Temperaturbeaufschlagung der Lanze 6 innerhalb des metallurgischen Gefässes kann es zu einem Verziehen der Lanze 6 kommen, so dass nach einer gewissen Betriebszeit die an der Lanze 6 vorgesehene Sondenhalterung 13 nicht mehr genau fluchtend über der mittels des in Arbeitsposition A geschwenkten Greifers 3 in Aufschiebeposition gehaltenen Sonde 4 steht.

Um trotzdem das Aufschieben der Sonde 4 auf den Haltestab sicherstellen zu können, ist zweckmässig am Greifer 3 oberhalb desselben eine Zentriereinrichtung 44 vorgesehen. Diese Zentriereinrichtung weist, wie aus Fig. 6 ersichtlich ist, zwei sichelförmig gestaltete und zur Mittelachse 45 symmetrisch angeordnete Fangarme 46 auf, die um eine in der Mittelachse 45 liegende Schwenkachse 47 aus einer in Fig. 6 mit vollen Linien dargestellten Ruheposition B in eine in Fig. 6 mit strichlierten Linien dargestellte Fangposition C schwenkbar sind.

Die für die beiden übereinanderliegenden Fangarme 46 gemeinsame Schwenkachse 47 ist in einem in horizontaler Richtung 48 senkrecht zur Lanzenachse 18 entlang von Führungssäulen 49, 50, die an einem ebenfalls horizontal und normal zur Achse 18 der Lanze 6 verschiebbaren Schlitten 51 befestigt sind, bewegbaren Führungsklotz 52 vorgesehen. Zur Horizontalführung des Schlittens 51 dienen in einem Rahmen 53 angeordnete Führungen 54, die parallel zu den Führungssäulen 49, 50 liegen.

Ein Linearantrieb, der als Druckmittelzylinder 55 ausgebildet ist, ist einerseits am Rahmen 53 und andererseits - mit seiner Kolbenstange 56 - am Schlitten 51 angelenkt. Die Fangarme 46 sind mittels an ihnen im Abstand von der Schwenkachse 47 angelenkter Laschen 57 mit dem Schlitten 51 verbunden.

Zwischen dem Schlitten 51 und dem Führungsklotz 52 ist eine Schraubenfeder 58 gespannt, zu deren Lagesicherung ein sich parallel zu den Führungen 54 bzw. Führungssäulen 49, 50 erstreckender Bolzen 59 dient.

Die Funktion der Einrichtung ist folgende:

Wird der Linearantrieb 55 beaufschlagt, so wird der in Ruheposition befindliche Schlitten 51 entlang der Führungen 54 in Richtung zur Lanze 6 verschoben, wobei der Führungsklotz 52, der durch die Schraubenfeder 58 in Distanz zum Schlitten 51 gehalten wird, synchron mitverschoben wird. Dadurch gelangen die Fangarme 46 im offenen Zustand in eine Position oberhalb des Greifers 3, d.h. der Zangenbackenpaare 15.

Die Bewegung des Führungsklotzes 52 wird, sobald er sich am vorderen Ende des Rahmens 53 befindet, durch einen nicht dargestellten Anschlag gestoppt. Der Schlitten 51 bewegt sich jedoch vom Linearantrieb 55 angetrieben weiter, so dass die Distanz zwischen Schlitten 51 und Führungsklotz 52 unter Zusammendrückung der Schraubenfeder 58 verringert wird. Infolge der zwischen dem Schlitten 51 und dem Führungsklotz 52 stattfindenden Relativbewegung werden die Fangarme 46 mittels der Laschen 57 in Fangposition C bewegt.

Wie aus Fig. 6 ersichtlich ist, ist die Form der Fangarme 46 so ausgebildet, dass die einander übergreifenden Fangarme 46 in Fangposition C einen dem Querschnitt des zu fangenden Teiles angepassten freien Raum 60 umschliessen. Gemäss dem dargestellten Ausführungsbeispiel ist dieser freie Raum etwa kreisförmig und entspricht hinsichtlich seines Durchmessers dem Durchmesser des an der Lanze 6 vorgesehenen Haltestabes 13 für die Sonden 4.

Während des Aufschiebens der Sonde 4 auf den Haltestab 13 wird ab einer bestimmten Höhe der Aufschiebebewegung der Linearantrieb 55 zur Einleitung der Rückzugsbewegung der Fangarme 46 beaufschlagt. Dabei bewegt sich zunächst lediglich der Schlitten 51 entlang der Führungen 54 von der Achse 18 der Lanze 6 weg wogegen der Führungsklotz infolge der Spannkraft der Feder 58 zunächst in seiner vordersten Position verbleibt. Während dieses Zurückbewegens des Schlittens 51 werden die Fangarme 46 geöffnet. Ist das Ende des Federweges durch den Schlitten 51 erreicht, bewegen sich der Schlitten 51 und der Führungsklotz 52 synchron bis zum Hubende des Linearantriebes 55 zurück, wodurch die Fangarme 46 in die Position B zurückgezogen werden und den Raum oberhalb des Greifers nicht mehr beschränken.

**Patentansprüche**

1. Vorrichtung (1) zum Wechseln von Mess- und/oder Probenahmesonden (4), die auf eine am unteren Ende einer vertikal beweglichen Lanze (6) angeordnete Halterung (13) unter Reibschlusskontakt aufschiebbar sind, mit einem die Sonde (4) festklemmenden, an einem Arm (2) der Vorrichtung angeordneten Greifer (3) der mittels des Armes aus einer Arbeitsposition (A) unterhalb der Lanze (6) in eine seitlich daneben befindliche Position bringbar ist, dadurch gekennzeichnet, dass der Greifer (3) an dem Arm (2) kardanisch gelagert ist.

2. Vorrichtung nach Anspruch 1, dadurch gekennzeichnet, dass der Schnittpunkt (26) der Achsen (23, 28) der kardanischen Lagerung bei einem in Arbeitsposition (A) befindlichen Greifer (3) in der Achse (18) der Lanze (6) liegt.

3. Vorrichtung nach Anspruch 1 oder 2, dadurch gekennzeichnet, dass der Greifer (3) aus seiner Ruhelage heraus gegen Rückstellkräfte kardanisch verschwenkbar ist, wobei die Rückstellkräfte entweder durch elastische Mittel (32, 33, 40) oder durch Anordnung des Schwerpunktes des Greifers (3) unterhalb des Schnittpunktes (26) der Achsen (23, 28) der kardanischen Lagerung hervorgerufen sind.

4. Vorrichtung nach den Ansprüchen 1 bis 3, dadurch gekennzeichnet, dass die Halterung (13) im Abstand (43) unterhalb des Schnittpunktes (26) der Achsen (23, 28) der kardanischen Lagerung mit der Sonde (4) in Reibschlusskontakt gelangt.

5. Vorrichtung nach den Ansprüchen 1 bis 4, mit einer im Abstand oberhalb des in Arbeitsposition (A) befindlichen Greifers (3) angeordneten Zentriereinrichtung (44), wobei die Zentriereinrichtung (44) zwei Fangarme (46) aufweist, die aus einer Ruheposition (B) seitlich der Halterung (13) in eine die Halterung (13) fluchtend oberhalb des in Arbeitsposition (A) befindlichen Greifers (3) festlegende Fangposition (C) bewegbar sind.

6. Vorrichtung nach Anspruch 5, dadurch gekennzeichnet, dass die Fangarme (46) als übereinanderliegende sichelförmige Arme ausgebildet sind und aus einer geöffneten Ruheposition (B) in eine die Halterung (13) umschliessende Fangposition (C) schwenkbar sind, und zwar unter Verkleinerung des von den Fangarmen (46) umschlossenen Raumquerschnittes bis auf den Querschnitt (60) der Halterung (13).

7. Vorrichtung nach Anspruch 5 oder 6, dadurch gekennzeichnet, dass die Zentriereinrichtung (44) an einem den Greifer (3) bewegenden Arm (2) montiert ist.

## Claims

1. An arrangement (1) for exchanging measuring and/or sampling probes (4) capable of being slipped on, with friction-tight contact, to a holding means (13) arranged on the lower en of a vertically movable lance (6), comprising a grab (3) clamping the probe (4) and arranged on an arm (2) of the arrangement, which grab is movable by means of the arm, from a working position (A) below the lance (6) into a position laterally therebeside, characterised in that the grab (3) is cardanically mounted on the arrangement (1).

2. An arrangement according to claim 1, characterised in that the point of intersection (26) of the axes (23, 28) of the cardanic mounting is located on the axis (18) of the lance (6) with a grab (3) being in the operation position (A).

3. An arrangement according to claim 1 or 2, characterised in that the grab (3) is cardanically pivotable from its resting position against restoring forces, the restoring forces being produced either by elastic means (32, 33, 40) or by arranging the center of gravity of the grab (3) below the point of intersection (26) of the axes (23, 28) of the cardanic mounting.

4. An arrangement according to claims 1 to 3, characterised in that the holding means (13) gets into friction-tight contact with the probe (4) at a distance (43) below the point of intersection (26) of the axes (23, 28) of the cardanic mounting.

5. An arrangement according to claims 1 to 4, comprising a centering means (44) arranged at a distance above the grab (3) being in the operation position (A), wherein the centering means (44) includes two catch arms (46) movable from a resting position (B) lateral of the holding means (13) into a catch position (C) fixing the holding means (13) in alignment above the grab (3) being in the operation position (A).

6. An arrangement according to claim 5, characterised in that the catch arms (46) are designed as superposed sickleshaped armes and are pivotable from an opened resting position (B) into a catch position (C) emoracing the holding means (13), i.e., by reducing the cross section of the space enclosed by the catch arms (46) to the cross section (60) of the holding means (13).

7. An arrangement according to claim 5 or 6, characterised in that the centering means (44) is mounted to an arm (2) moving the grab (3).

## Revendications

1. Dispositif (1) pour changer des sondes de mesure et/ou de prélèvement d'échantillons emboîtables, avec établissement d'une liaison par contact et frottement, sur un organe de maintien (13) agencé à l'extrémité inférieure d'une lance (6) mobile verticalement, ce dispositif comportant un moyen de préhension (3) serrant la sonde (4) et agencé sur un bras (2) du dispositif, le moyen de préhension pouvant etre amené, au moyen du bras, d'une position de travail (A) en dessous de la lance (6) à une position latérale proche, caractérisé par le fait que le moyen de préhension (3) est monté à la Cardan sur le bras (2).

2. Dispositif selon revendications 1, caractérisé par le fait que le point d'intersection (26) des axes (23, 28) du montage à la Cardan se trouve sur l'axe (18) de la lance (6) lorsque le moyen de préhension (3) se trouve en position de travail (A).

3. Dispositif selon revendication 1 ou 2, caractérisé par le fait que le moyen de préhension (3) peut être écarté angulairement, à la Cardan, de sa position de repos, contre des forces de rappel, lesquelles sont produites soit par des moyens élastiques (32, 33, 40), soit par agencement du centre de gravité du moyen de préhension (3) en dessous du point d'intersection (26) des axes (23, 28) du montage à la Cardan.

4. Dispositif selon les revendications 1 à 3, caractérisé par le fait que l'organe de maintien (13) vient en contact, établissant une liaison par frottement, avec la sonde (4) à distance (43) en dessous du point d'intersection (26) des axes (2, 28) du montage à la Cardan.

5. Dispositif selon les revendications 1 à 4, avec un dispositif de centrage (44) agencé à distance au-dessus du moyen de préhension (3) se trouvant en position de travail (A), le dispositif de centrage (44) présentant deux bras capteurs (46) qui peuvent être déplacés d'une position de repos (B) latérale à l'organe de maintien (13) à une position de tenue (C) maintenant l'organe de maintien (13) aligné audessus du moyen de préhension (3) se trouvant en position de travail (A)

6. Dispositif selon revendication 5, caractérisé par le fait que les bras capteurs (46) sont réalisés sous forme de bras superposés en forme de faucille et peuvent être déplacés par pivotement, d'une position de repos ouverte (B) à une position de tenue (C) enfermant l'organe de maintien (13), et cela en réduisant la portion d'espace entourée par les bras capteurs (46) jusqu'à la section droite (60) de l'organe de maintien (13).

7. Dispositif selon revendication 5 ou 6, caractérisé par le fait que le dispositif de centrage (44) est monté sur un bras (2) déplaçant le moyen de préhension (3).

FIG. 1

FIG. 2

# FIG. 3

FIG. 5

FIG. 4

FIG. 6